(19) Europäisches Patentamt
European Patent Office
Office européen des brevets

(11) Veröffentlichungsnummer : **0 090 258**
**B1**

(12) # EUROPÄISCHE PATENTSCHRIFT

(45) Veröffentlichungstag der Patentschrift : **29.07.87**

(51) Int. Cl.[4] : **C 07 D215/48**, C 07 D401/12, A 01 N 43/42

(21) Anmeldenummer : **83102589.5**

(22) Anmeldetag : **16.03.83**

(54) **3-Chlor-8-cyano-chinoline, Verfahren zu ihrer Herstellung und ihre Verwendung zur Bekämpfung unerwünschten Pflanzenwuchses.**

(30) Priorität : **25.03.82 DE 3210979**

(43) Veröffentlichungstag der Anmeldung : **05.10.83 Patentblatt 83/40**

(45) Bekanntmachung des Hinweises auf die Patenterteilung : **29.07.87 Patentblatt 87/31**

(84) Benannte Vertragsstaaten : **DE FR GB**

(56) Entgegenhaltungen :
**EP-A- 0 060 429**
**DE-A- 2 437 297**
**GB-A- 1 419 788**

(73) Patentinhaber : **BASF Aktiengesellschaft**
**Carl-Bosch-Strasse 38**
**D-6700 Ludwigshafen (DE)**

(72) Erfinder : **Hagen, Helmut, Dr.**
**Max-Slevogt-Strasse 17E**
**D-6710 Frankenthal (DE)**
Erfinder : **Markert, Juergen, Dr.**
**Am Speyerweg 26**
**D-6704 Mutterstadt (DE)**
Erfinder : **Kohler, Rolf-Dieter, Dr.**
**Amselweg 3**
**D-6803 Edingen-Neckarhausen (DE)**
Erfinder : **Wuerzer, Bruno, Dr. Dipl.-Landwirt**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 090 258 B1

Jouve, 18, rue St-Denis, 75001 Paris, France

## Beschreibung

Die Erfindung betrifft 3-Chlor-8-cyano-chinoline, Verfahren zu ihrer Herstellung, Herbizide, die diese Verbindungen als Wirkstoffe enthalten, sowie ein Verfahren zur Bekämpfung unerwünschten Pflanzenwuchses mit diesen Wirkstoffen.

Chinolinverbindungen mit schwachen herbiziden Eigenschaften sind aus DE-OS 23 22 143 und US-PS 2 661 276 bekannt.

Es wurde gefunden, daß 3-Chlor-8-cyano-chinoline der Formel

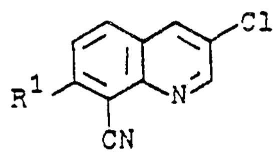

(I)

in der $R^1$ für Chlor, Brom oder die Gruppe $—NR^2R^3$, wobei $R^2$ und $R^3$ gleich oder verschieden sind und Wasserstoff, $C_1$-$C_8$-Alkyl, $C_2$-$C_6$-Alkenyl, $C_1$-$C_6$-Hydroxyalkyl, $C_1$-$C_6$-(ω-Dialkylamino)-alkyl, Formyl, Cyclohexyl, Phenyl, Pyridyl oder zusammen den Tetramethylen- oder den Pentamethylenrest bedeuten, wobei eine Methylengruppe in diesen Resten durch ein Sauerstoff- oder Stickstoffatom oder die $—N(CH_3)$-Gruppe ersetzt sein kann, steht, eine stärkere herbizide Wirkung haben als bekannte Chinolinderivate.

In Formel I kann $R^1$ außer Chlor oder Brom die Gruppe $—NR^2R^3$ bedeuten. Dabei stehen $R^2$ und $R^3$ für Wasserstoff, unverzweigte oder verzweigte Alkylreste mit 1 bis 8, vorzugsweise 1 bis 4 Kohlenstoffatomen, unverzweigte oder verzweigte Alkenylreste mit 2 bis 6, vorzugsweise 2 bis 4 Kohlenstoffatomen, unverzweigte oder verzweigte Hydroxyalkylreste mit 1 bis 6, vorzugsweise 1 bis 4 Kohlenstoffatomen, unverzweigte oder verzweigte ω-Dialkylamino-alkylreste mit 1 bis 6 Kohlenstoffatomen im Alkylrest und 1 bis 4 Kohlenstoffatomen im Dialkylaminorest, Formyl, Cyclohexyl, Phenyl oder Pyridyl. $R^2$ und $R^3$ können auch zusammen einen Tetramethylen- oder Pentamethylenrest bilden, wobei eine der Methylengruppen durch Sauerstoff, Stickstoff oder die $—N(CH_3)$-Gruppe ersetzt sein kann. Beispiele für solche Reste sind Amino, Dimethylamino, Di-n-butylamino, n-Butylamino, i-Butylamino, Di-n-propylamino, N-Ethyl-N-n-butylamino, i-Propylamino, N-Methyl-N-phenylamino, Cyclohexylamino, Di-cyclohexylamino, Ethylamino, n-Propylamino, n-Hexylamino, n-Octylamino, n-Pentylamino, Allylamino, Diallylamino, (4-Diethylamino-n-butyl)-amino, (3-Dimethylamino-2,2-dimethyl-n-propyl)-amino, 2-Hydroxyethyl-amino, Dimethallylamino, Diethylamino, Diisopropylamino, Methylamino, Di-(2-hydroxyethyl)-amino, Morpholino, Tetramethylenimino, Pentamethylenimino, 4-Methyl-piperazin-1-yl, Piperidin-1-yl, Pyrrolidin-1-yl. Bevorzugt sind Verbindungen der Formel I, wobei $R^1$ für den Rest $—NR^2R^3$ steht, wobei $R^2$ Wasserstoff und $R^3$ $C_1$-$C_8$-Alkyl, insbesondere $C_1$-$C_4$-Alkyl, oder Cyclohexyl bedeuten.

Verbindungen der Formel I, bei denen $R^1$ Chlor oder Brom bedeutet, erhält man in an sich üblicher Weise durch Umsetzung von 3-Chlor-8-dichlormethyl-chinolinderivaten der Formel

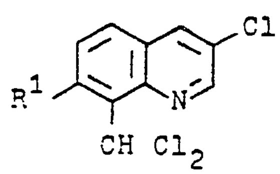

(III)

in der $R^1$ Chlor oder Brom bedeutet, mit Hydroxylaminhydrochlorid und Natriumformiat in 100 %iger Ameisensäure bei 100 °C. Die Reaktionsdauer beträgt 10 bis 20 Stunden.

Verbindungen der Formel I, bei denen $R^1$ eine $—NR^2R^3$-Gruppe bedeutet, erhält man durch Umsetzung von 3,7-Dichlor-8-cyano-chinolin mit einem Amin der Formel $HNR^2R^3$, in der $R^2$ und $R^3$ die obengenannten Bedeutungen haben. Die Umsetzung wird in an sich bekannter Weise gegebenenfalls in Gegenwart eines inerten organischen Lösungsmittels bei einer Temperatur im Bereich zwischen 60 und 180 °C, vorzugsweise 80 bis 110 °C, durchgeführt. Die Reaktionszeit beträgt 1 bis 30, üblicherweise 10 bis 20 Stunden. Geeignete Lösungsmittel sind Dimethylformamid, Dimethylsulfoxid, Alkohole, wie Methanol, gegebenenfalls substituierte Aromaten, wie Toluol, Xylole, Chlorbenzol, Dichlorbenzole.

3-Chlor-7-amino-8-cyano-chinolin erhält man durch Umsetzen von 3,7-Dichlor-8-cyano-chinolin mit flüssigem Ammoniak, gegebenenfalls in Gegenwart eines Lösungsmittels, vorzugsweise eines Alkohols, im Autoklaven.

Die folgenden Beispiele erläutern die Herstellung der 3-Chlor-8-cyano-chinoline der Formel I.

### Beispiel 1

22,3 g 3,7-Dichlor-8-cyanochinolin und 26 g Di-n-butylamin werden in 300 ml Methoxypropanol 12 Stunden unter Rückfluß erhitzt. Die Lösung wird abgekühlt, der ausgefallene Feststoff abgesaugt und aus einem Gemisch aus Ligroin und Toluol umkristallisiert. Man erhält 26 g 3-Chlor-8-cyano-7-di-n-butylaminochinolin vom Schmelzpunkt 112 °C ; Ausbeute : 82,5 % d. Th.

Beispiel 2

44,5 g 3,7-Dichlor-8-cyanochinolin werden in 200 g n-Butylamin 14 Stunden auf 80 °C erhitzt. Die Lösung wird auf Eiswasser gegossen und zweimal mit je 100 ml Methylenchlorid ausgezogen, die vereinigten Auszüge mit $Na_2SO_4$ getrocknet und eingeengt. Der ölige Rückstand wird mit Ether behandelt und der ausgefallene Feststoff abgesaugt. Es werden 39 g 7-n-Butylamino-3-chlor-8-cyanochinolin vom Schmelzpunkt 89 °C erhalten ; Ausbeute : 75 % d. Th.

Beispiel 3

110 g 3,7-Dichlor-8-cyanochinolin werden in 500 ml Dimethylformamid 20 Stunden am Rückfluß erhitzt. Man kühlt die Lösung ab, setzt die Hälfte der Volumenmenge an Wasser zu und saugt den ausgefallenen Feststoff ab, wobei 100 g 3-Chlor-8-cyano-7-dimethylaminochinolin vom Schmelzpunkt 174 °C erhalten werden ; Ausbeute : 87 % d. Th.

Beispiel 4

110 g 3,7-Dichlor-8-cyano-chinolin, 500 ml Methanol und 500 ml flüssiger Ammoniak werden im Autoklaven 10 Stunden auf 150 °C erhitzt. Nach dem Entspannen wird der Austrag abgesaugt und der Feststoff in Gegenwart von Aktivkohle aus Methylglykol umkristallisiert. Es werden 54 g 7-Amino-3-chlor-8-cyanochinolin vom Schmelzpunkt 246 °C erhalten ; Ausbeute : 53 % d. Th.

Beispiel 5

177 g 7-Chlor-8-methylchinolin und 1 g Azo-bisisobutyronitril werden in 100 ml Dichlorbenzol auf 140 °C erhitzt. Während des Chloreinleitens wird die Temperatur auf ca. 175 °C erhöht. Die Reaktion wird gaschromatographisch überwacht. Nach beendeter Reaktion wird die Lösung mit Stickstoff gespült, der größte Teil des Lösungsmittels abdestilliert, der ausgefallene Feststoff abgesaugt und mit Petrolether gewaschen. Ausbeute : 255 g 3,7-Dichlor-8-dichlormethylchinolin (80 % d. Th.) ; Schmelzpunkt : 154 °C.

28,1 Gew. Teile des so erhaltenen 3,7-Dichlor-8-dichlormethylchinolin, 6,95 Gew. Teile Hydroxylaminohydrochlorid und 13,6 Gew. Teile Natriumformiat werden in 200 ml Ameisensäure und 60 ml Wasser 12 Stunden bei 100 °C gerührt. Die Reaktionslösung wird auf Eis gegossen, der ausgefallene Feststoff abgesaugt, mit Wasser neutral gewaschen und getrocknet. Es werden 19 Gew. Teile 3,7-Dichlor-8-cyanochinolin vom Schmp. 222 °C erhalten ; Ausbeute : 78,5 % d. Th.

Analog können folgende Verbindungen der Formel I hergestellt werden :

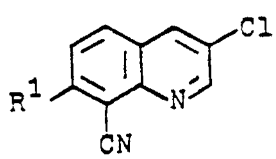

(I)

| Nr. | $R^1$ | Schmp. [°C] |
|---|---|---|
| 6 | $-NH-i-C_4H_9$ | 97 |
| 7 | $-N(C_2H_5)_2$ | 126 |
| 8 | $-N(n-C_3H_7)_2$ | 120 |
| 9 | 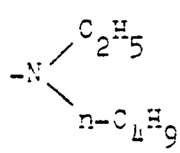 | 110 |
| 10 | 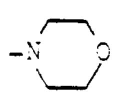 | 158 |

(Fortsetzung)

| Nr. | $R^1$ | Schmp. [°C] |
|---|---|---|
| 11 | $-N(CH_2-CH=CH_2)_2$ | 120 |
| 12 | 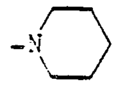 | 125 |
| 13 | 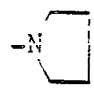 | 252 |
| 14 | 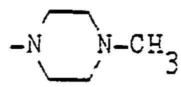 | 136 |
| 15 | $NH-i-C_3H_7$ | 139 |
| 16 | N-Methyl-N-phenylamino | |
| 17 | Cyclohexylamino | 161 |
| 18 | Dicyclohexylamino | |
| 19 | $NH-C_2H_5$ | |
| 20 | $NH-n-C_3H_7$ | 100 |
| 21 | $NH-n-C_6H_{13}$ | |
| 22 | $NH-n-C_8H_{17}$ | 105 |
| 23 | $NH-(CH_2)_4-N(C_2H_5)_2$ | |
| 24 | $NH-CH_2-C(CH_3)_2-CH_2-N(CH_3)_2$ | 122 |
| 25 | $NH-CH_2CH_2OH$ | 172 |
| 26 | $NH-n-C_5H_{11}$ | 103 |
| 27 | $NH-CH_2-CH=CH_2$ | 170 |

Die Verbindungen der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen

kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile des Wirkstoffs Nr. 1 mit 10 Gewichtsteilen N-Methyl-α-pyrrolidon und erhält eine Lösung, die zur Anwendung in Form kleinster Tropfen geeignet ist.

II. 20 Gewichtsteile des Wirkstoffs Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsaure-N-mono-ethanolamid, 5 Gewichtsteilen Calciumsalz der Dodecyl-benzosulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile des Wirkstoffs Nr. 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile des Wirkstoffs Nr. 4 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile des Wirkstoffs Nr. 16 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile des Wirkstoffs Nr. 14 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile des Wirkstoffs Nr. 14 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 5 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls vermischt. Man erhält eine stabile ölige Dispersion.

Die Applikation der Wirkstoffe bzw. der Mittel kann im Vorauflaufverfahren oder bei Nachauflaufanwendung erfolgen. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Zielpflanzen und Wachstumsstadium 0,05 bis 5 kg/ha und mehr, vorzugsweise 0,1 bis 3 kg/ha.

Die herbizide Wirkung von Verbindungen der Formel I wird durch Gewächshausversuche gezeigt :

Als Kulturgefäße dienten Plastikblumentöpfe mit 300 $cm^3$ Inhalt und lehmigem Sand mit etwa 1,5 % Humus als Substrat. Die Samen der Testpflanzen wurden nach Arten getrennt flach eingesät.

Zum Zwecke der Nachauflaufbehandlung zog man die Testpflanzen je nach Wuchsform erst bis zu einer Wuchshöhe von 3 bis 15 cm an und behandelte sie danach. Die Wirkstoffe wurden hierbei in Wasser als Verteilungsmittel suspendiert oder emulgiert und mittels fein verteilender Düsen gespritzt. Die Reispflanzen wurden in einem mit Torfmull angereicherten Substrat angezogen. Zur Nachauflaufbehandlung wurden entweder direkt gesäte und in den gleichen Gefäßen aufgewachsene Pflanzen ausgewählt, oder sie wurden erst als Keimpflanzen getrennt angezogen und einige Tage vor der Behandlung in die Versuchsgefäße verpflanzt. Die Aufwandmenge für die Nachauflaufbehandlung betrug 2,0 und 3,0 kg Wirkstoff/ha.

Die Versuchsgefäße wurden im Gewächshaus aufgestellt, wobei für wärmeliebende Arten wärmere Bereiche (20 bis 35 °C) und für solche gemäßigter Klimate 10 bis 25 °C bevorzugt wurden. Die Versuchsperiode erstreckte sich bis zu 4 Wochen. Während dieser Zeit wurden die Pflanzen gepflegt, und ihre Reaktion auf die einzelnen Behandlungen wurde ausgewertet. Bewertet wurde nach einer Skala von 0 bis 100. Dabei bedeutet 0 keine Schädigung oder normaler Auflauf und 100 kein Aufgang der Pflanzen bzw. völlige Zerstörung zumindest der oberirdischen Teile.

Die in den Gewächshausversuchen verwendeten Pflanzen setzten sich aus folgenden Arten zusammen : Amaranthus spp. (zurückgekrümmter Fuchsschwanz), Echinochloa crus-galli (Hühnerhirse), Ipomoea spp. (Prunkwinde), Lamium purpureum (Rote Taubnessel), Oryza sativa (Reis), Sesbania exaltata (Turibaum), Solanum nigrum (Schwarzer Nachtschatten).

Bei der Prüfung auf herbizide Wirkung bei Nachauflaufanwendung bei Aufwandmengen von 3,0 kg Wirkstoff/ha zeigen beispielsweise Verbindungen Nr. 15, 17, 20 und 26 eine gute herbizide Wirkung grasartige Pflanzen, während die Verbindung Nr. 2 beispielsweise mit 2,0 kg/ha breitblättrige unerwünschte Pflanzen gut bekämpft.

Je nach Applikationsverfahren, Anwendungsdosis und vorherrschender Verunkrautung können die Verbindungen der Formel I bzw. sie enthaltende herbizide Mittel beispielsweise in folgenden Kulturen eingesetzt werden :

| Botanischer Name | Deutscher Name |
|---|---|
| Allium cepa | Küchenzwiebel |
| Ananas Comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor-Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |

| Botanischer Name | Deutscher Name |
|---|---|
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| etroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die 3-Chlor-8-cyanochinoline der Formel I bzw. die sie enthaltenden herbiziden Mittel allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Patentansprüche

1. 3-Chlor-8-cyano-chinoline der Formel

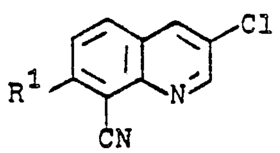

(I)

in der $R^1$ für Chlor, Brom oder die Gruppe $-NR^2R^3$, wobei $R^2$ und $R^3$ gleich oder verschieden sind und Wasserstoff, $C_1$-$C_8$-Alkyl, $C_2$-$C_6$-Alkenyl, $C_1$-$C_6$-Hydroxyalkyl, $C_1$-$C_6$-($\omega$-Dialkylamino)-alkyl mit 1 bis 4 C-Atomen im Dialkylaminorest, Formyl, Cyclohexyl, Phenyl, Pyridyl oder zusammen den Tetramethylen- oder den Pentamethylenrest bedeuten, wobei eine Methylengruppe in diesen Resten durch Sauerstoff oder Stickstoff oder die $-N(CH_3)$-Gruppe ersetzt sein kann, steht.

2. 3-Chlor-8-cyano-chinoline der Formel I gemäß Anspruch 1, dadurch gekennzeichnet, daß $R^1$ für die Gruppe $-NR^2R^3$, wobei $R^2$ Wasserstoff und $R^3$ $C_1$-$C_8$-Alkyl oder Cyclohexyl bedeuten, steht.

3. 7-n-Butylamino-3-chlor-8-cyano-chinolin.

4. Verfahren zur Herstellung von 3-Chlor-8-cyano-chinolinen der Formel I gemäß Anspruch 1, in der $R^1$ die Gruppe $-NR^2R^3$ bedeutet, dadurch gekennzeichnet, daß man 3,7-Dichloro-8-cyano-chinolin in an sich bekannter Weise mit einem Amin der Formel

$$HNR^2R^3 \quad \text{(II)},$$

in der $R^2$ und $R^3$ die im Anspruch 1 genannten Bedeutungen haben, gegebenenfalls in Gegenwart eines inerten organischen Lösungsmittels bei einer Temperatur im Bereich zwischen 60 und 180 °C umsetzt.

5. Verfahren zur Herstellung von 3-Chlor-8-cyano-chinolinen der Formel I gemäß Anspruch 1, in der $R^1$ Chlor oder Brom bedeutet, dadurch gekennzeichnet, daß man ein 3-Chlor-8-dichlormethylchinolinderivat der Formel

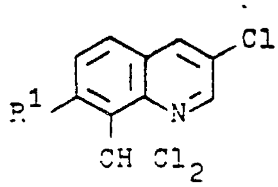

(III)

in der $R^1$ Chlor oder Brom bedeutet, in an sich bekannter Weise mit Hydroxylaminhydrochlorid und Natriumformiat in 100 %iger Ameisensäure bei 100 °C umsetzt.

6. Herbizid, enthaltend ein 3-Chlor-8-cyano-chinolin der Formel I gemäß Anspruch 1.

7. Herbizid, enthaltend inerte Zusatzstoffe und ein 3-Chlor-8-cyano-chinolin der Formel I gemäß Anspruch 1.

8. Herbizid, enthaltend inerte Zusatzstoffe und ein 3-Chlor-8-cyano-chinolin gemäß Anspruch 2.

9. Herbizid, enthaltend inerte Zusatzstoffe und 7-n-Butyl-amino-3-chlor-8-cyano-chinolin.

10. Verfahren zur Bekämpfung unerwünschten Pflanzenwuchses, dadurch gekennzeichnet, daß man die Pflanzen und/oder ihren Standort mit einer herbizid wirksamen Menge eines 3-Chlor-8-cyano-chinolins der Formel I gemäß Anspruch 1 behandelt.

## Claims

1. A 3-chloro-8-cyanoquinoline of the formula

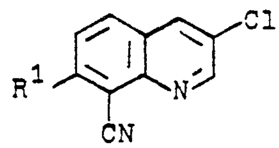

(I)

where $R^1$ is chlorine, bromine or $-NR^2R^3$, $R^2$ and $R^3$ being identical or different and each denoting hydrogen, $C_1$-$C_8$-alkyl, $C_2$-$C_6$-alkenyl, $C_1$-$C_6$-hydroxyalkyl, ω-dialkylamino-$C_1$-$C_6$-alkyl, where dialkylamino is of 1 to 4 carbon atoms, formyl, cyclohexyl, phenyl or pyridyl, or together denoting tetramethylene or pentamethylene, one methylene group in these radicals being optionally replaced by oxygen, nitrogen or $-N(CH_3)$.

2. A 3-chloro-8-cyanoquinoline of the formula I as claimed in claim 1, where $R^1$ is $-NR^2R^3$, $R^2$ denoting hydrogen and $R^3$ $C_1$-$C_8$-alkyl or cyclohexyl.

3. 7-n-Butylamino-3-chloro-8-cyanoquinoline.

4. A process for the preparation of a 3-chloro-8-cyanoquinoline of the formula I as claimed in claim 1, where $R^1$ is $-NR^2R^3$, wherein a 3-chloro-8-dichloromethylquinoline is reacted in a conventional manner with an amine of the formula

$$HNR^2R^3 \qquad (II),$$

where $R^2$ and $R^3$ have the meanings given in claim 1, in the presence or absence of an inert organic solvent at a temperature of from 60 to 180 °C.

5. A process for the preparation of a 3-chloro-8-cyanoquinoline of the formula I as claimed in claim 1, where $R^1$ is chlorine or bromine, wherein a 3-chloro-8-dichloro-methylquinoline derivative of the formula

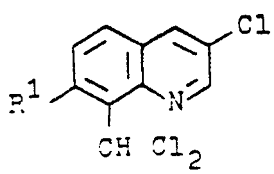

(III)

where $R^1$ is chlorine or bromine, is reacted in a conventional manner with hydroxylamine hydrochloride and sodium formate in 100 % strength formic acid at 100 °C.

6. A herbicide containing a 3-chloro-8-cyanoquinoline of the formula I as claimed in claim 1.

7. A herbicide containing inert additives and a 3-chloro-8-cyanoquinoline of the formula I as claimed in claim 1.

8. A herbicide containing inert additives and a 3-chloro-8-cyanoquinoline as claimed in claim 2.

9. A herbicide containing inert additives and 7-n-butylamino-3-chloro-8-cyanoquinoline.

10. A process for combating the growth of unwanted plants, wherein the plants and/or their location are treated with a herbicidally effective amount of a 3-chloro-8-cyanoquinoline of the formula I as claimed in claim 1.

## Revendications

1. 3-chloro-8-cyano-quinoline de formule

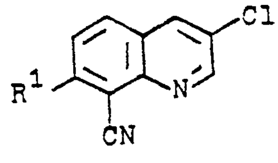

(I)

dans laquelle $R^1$ représente chlore, brome ou le groupe $-NR^2R^3$, $R^2$ et $R^3$ étant identiques ou différents et représentant hydrogène, alkyle en $C_1$-$C_8$, alcényle en $C_2$-$C_6$, hydroxyalkyle en $C_1$-$C_6$, ω-dialkylamino-alkyle en $C_1$-$C_6$, avec 1 à 4 atomes C dans le reste dialkylamino, formyle, cyclohexyle, phényle, pyridyle ou, ensemble, le reste tétraméthylène ou pentaméthylène, un groupe méthylène dans ces restes pouvant être remplacé par oxygène ou azote ou par le groupe $-N(CH_3)$.

2. 3-chloro-8-cyano-quinoline de formule I selon la revendication 1, caractérisé par le fait que $R^1$ est mis pour le groupe $-NR^2R^3$, $R^2$ représentant hydrogène et $R^3$, alkyle-$C_1$-$C_8$ ou cyclohexyle.

3. 7-n-butylamino-3-chloro-8-cyano-quinoline.

4. Procédé de préparation de 3-chloro-8-cyano-quinolines de formule I selon la revendication 1, dans laquelle $R^1$ représente le groupe $-NR^2R^3$, caractérisé par le fait qu'on fait réagir de la 3,7-dichloro-8-cyano-quinoline, de manière connue en soi, avec une amine de formule

$$HNR^2R^3 \quad (II),$$

dans laquelle $R^2$ et $R^3$ ont les significations indiquées dans la revendication 1, éventuellement en présence d'un solvant organique inerte, à une température comprise entre 60 et 180 °C.

5. Procédé de préparation de 3-chloro-8-cyano-quinolines de formule I selon la revendication 1, dans laquelle $R^1$ représente chlore ou brome, caractérisé par le fait que l'on fait réagir, à 100 °C, un dérivé de 3-chloro-8-dichloro-méthylquinoline de formule

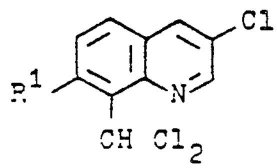

(III)

dans laquelle $R^1$ représente chlore ou brome, de manière connue en soi avec du chlorhydrate d'hydroxylamine et du formiate de sodium dans de l'acide formique à 100 %.

6. Herbicide, contenant un 3-chloro-8-cyano-quinoline de formule I selon la revendication 1.

7. Herbicide, contenant des additifs inertes et un 3-chloro-8-cyano-quinoline de formule I selon la revendication 1.

8. Herbicide, contenant des additifs inertes et un 3-chloro-8-cyano-quinoline de formule I selon la revendication 2.

9. Herbicide, contenant des additifs inertes et de la 7-n-butyl-amino-3-chloro-8-cyano-quinoline.

10. Procédé de lutte contre la croissance des plantes indésirables, caractérisé par le fait que l'on traite les plantes et/ou leur biotope avec une quantité efficace au point de vue herbicide d'une 3-chloro-8-cyano-quinoline de formule I selon la revendication 1.